# EUROPEAN PATENT APPLICATION

(11) **EP 4 718 067 A1**
(43) Date of publication of application: **01.04.2026**
(21) Application number: 23938154.4
(22) Date of filing: 05.09.2023
(51) Int. Cl.: G01N 27/327, G01N 33/66, G01N 33/64

(54) **MULTI-ANALYTE MONITORING SENSOR**

(30) Priority: 25.05.2023 CN 202310606611
(71) Applicant: SHENZHEN SISENSING CO., LTD., Shenzhen Guangdong 518110 (CN)
(72) Inventor: CHEN, Liguo, Shenzhen, Guangdong 518110 (CN); FANG, Junfei, Shenzhen, Guangdong 518110 (CN)
(74) Representative: Isern Patentes y Marcas S.L.
(86) International application number: PCT/CN2023/116865
(87) International publication number: WO 2024/239485

(57) **Abstract**

The present disclosure describes a multi-analyte monitoring sensor, which includes a substrate, a working electrode disposed on the substrate, an enzyme sensing layer disposed on the working electrode, and a polymer membrane layer disposed on the enzyme sensing layer; The working electrode includes a first working electrode and a second working electrode; The enzyme sensing layer includes a first analyte enzyme layer and a second analyte enzyme layer, the first analyte enzyme layer is disposed on the first working electrode and the second analyte enzyme layer is disposed on the second working electrode, the first analyte is glucose, and the second analyte is ketone; The polymer membrane layer is permeable to both the first analyte and the second analyte and covers the first analyte enzyme layer and the second analyte enzyme layer. Thus, a sensor with high sensitivity and simplified processing for continuous monitoring of blood glucose and blood ketones can be provided

## Description

### BACKGROUND OF INVENTION

### Field of Invention

The present disclosure relates substantially to the field of biosensors, specifically to a multi-analyte monitoring sensor.

### Description of Related Art

A biosensor is an analytical device that closely combines biomaterials, bio-derived materials, or biomimetic materials with optical, electrochemical, temperature, piezoelectric, magnetic, or micromechanical physicochemical sensors or sensing microsystems. It is typically used to rapidly detect certain specific chemical substances in the human body, such as glucose, ketone bodies, uric acid, and a series of amino acid compounds.

When too high blood glucose, lacking the insulin in the body, or on a ketogenic diet, the body will metabolize fat to produce ketone bodies. When the amount of ketone bodies exceeds the metabolic capacity of the liver, complications of diabetes can be easily led to. Therefore, the monitoring of blood ketone levels is also of great significance in diabetic patients.

Traditional methods for detecting blood ketones mainly include urine ketone test strip, blood ketone test strip, and biochemical analysis etc., which quantitatively or qualitatively detect the content of β-hydroxybutyric acid (referred to as β-BHB or hydroxybutyric acid), acetone, and acetoacetic acid in blood or urine, so as to reflect the level of ketone bodies. Typically, wearable acetone sensors detect acetone in the exhaled breath of diabetic patients, convert resistance changes into digital quantities, and send them to a display module to display the acetone concentration.

However, the response time of the aforementioned acetone sensors is usually around 50 seconds, resulting in a lag in acetone assessment; Moreover, when it is necessary to simultaneously monitor blood glucose and blood ketones, it is generally necessary to separately set up a blood glucose sensor to monitor blood glucose, which is inconvenient for use.

### SUMMARY OF INVENTION

The present disclosure is made in view of the above-mentioned situation, and its object is to provide a multi-analyte monitoring sensor capable of continuously monitoring both blood glucose and blood ketone at the same time.

For the above purposes, the present disclosure provides a multi-analyte monitoring sensor, the sensor includes a substrate, a working electrode disposed on the substrate, an enzyme sensing layer disposed on the working electrode, and a polymer membrane layer disposed on the enzyme sensing layer. The working electrode includes a first working electrode and a second working electrode; The enzyme sensing layer includes a first analyte enzyme layer and a second analyte enzyme layer, the first analyte enzyme layer is disposed on the first working electrode and the second analyte enzyme layer is disposed on the second working electrode, the first analyte is glucose, and the second analyte is ketone. The polymer membrane layer is permeable to both the first analyte and the second analyte and covers the first analyte enzyme layer and the second analyte enzyme layer.

**In** the multi-analyte monitoring sensor according to the present disclosure, the enzyme layers of two different analytes are disposed on the two working electrodes of the sensor, thereby the concentration levels of two different analytes in the tissue fluid can be detected respectively. The polymer membrane layer is permeable to both glucose and ketones at the same time, the first analyte enzyme layer and the second analyte enzyme layer are covered with the polymer membrane layer, resulting in restricting the permeation of both analytes, and facilitating the improvement of stability of the sensor; In addition, due to the small size of the biosensor, high precision is required for the coating on the surface of the sensor. Usually, the dipping and pulling method is used to cover the surface of the sensor with a membrane solution to form a membrane layer coating the sensor. Comparing to the scheme of separately disposing semi-permeable membranes permeable to glucose and ketones on the first analyte enzyme layer and the second analyte enzyme layer, the polymer membrane in the present disclosure may simultaneously permeate glucose and ketones, thereby simplifying the sensor's manufacturing process while maintaining its high sensitivity and accuracy. Thus, the sensor with a simple manufacturing process capable of continuously monitoring glucose and ketones simultaneously can be provided.

In addition, in the multi-analyte monitoring sensor according to the present disclosure, optionally, the polymer membrane layer includes a vinyl pyridine-based polymer, a cross-linking agent, and a modifier, wherein the mass fraction of the vinyl pyridine-based polymer in the polymer membrane layer is 80% to 100%. Thus, the formation of the biocompatible polymer membrane layer with limited permeability to glucose and ketones can be facilitated, the amount of glucose and ketones can be controlled within the linear range of the sensor through the polymer membrane, resulting in improving the sensitivity and accuracy of the sensor.

In addition, in the multi-analyte monitoring sensor according to the present disclosure, optionally, the polymer membrane layer includes a first membrane layer disposed successively on the enzyme sensing layer, a transition layer formed on the first membrane layer, and a biocompatible second membrane layer formed on the transition layer. Thus, the adhesive stability of the membrane layer can be improved, resulting in facilitating the improvement of stability of the sensor.

In addition, in the multi-analyte monitoring sensor according to the present disclosure, optionally, the first membrane layer is formed by a first type of polymer, the second membrane layer is formed by a second type of polymer, the transition layer is formed by a third type of polymer, wherein the third type of polymer is a copolymer formed by a first monomer identical or similar to the first type of polymer and a second monomer identical or similar to the second type of polymer. Thus, the adhesive stability and mechanical strength of the polymer membrane layer can be improved, resulting in improving the stability of the sensor.

In addition, in the multi-analyte monitoring sensor according to the present disclosure, optionally, the first membrane layer includes a vinyl pyridine-based polymer, the transition layer and the second membrane layer include a copolymer of vinyl pyridine and styrene. Thus, the improvement of membrane-forming property of the membrane layer can be facilitated, resulting in improving the sensitivity of the sensor.

In addition, in the multi-analyte monitoring sensor according to the present disclosure, optionally, the area of the first analyte enzyme layer is greater than the area of the second analyte enzyme layer. Since the amounts of glucose and ketone permeable through the polymer membrane are different, and generally, the amount of glucose is less than the amount of ketone, in this case, the area of the first analyte enzyme layer is configured to be greater than the area of the second analyte enzyme layer, the contact area between the first analyte enzyme layer and glucose can be increased, resulting in reducing the current difference between the two working electrodes as much as possible, enabling the current levels on the two working electrodes to be at the same level, reducing the interference of the electric field on the sensor system, and further improving the measurement accuracy of the sensor.

In addition, in the multi-analyte monitoring sensor according to the present disclosure, optionally, the area ratio of the first analyte enzyme layer to the second analyte enzyme layer is 1:1 to 1.5:1. Thus, the reaction sensitivity of the first analyte can be improved, resulting in a more accurate reflection of the concentration level of the analyte in the tissue fluid.

In addition, in the multi-analyte monitoring sensor according to the present disclosure, optionally, the first analyte enzyme layer and/or the second analyte enzyme layer are/is in a linear shape. In this case, compared to the scheme of multi-point discrete arrangement, a larger area of the enzyme layer can be arranged on the limited area of the working electrode, resulting in improving the sensitivity of the sensor.

In addition, in the multi-analyte monitoring sensor according to the present disclosure, optionally, The second analyte enzyme layer includes hydroxybutyrate dehydrogenase, diaphorase, coenzyme, and an electron mediator, in the second analyte enzyme layer, the mass fraction of hydroxybutyrate dehydrogenase is 10% to 20%, the mass fraction of diaphorase is 5% to 20%, the mass fraction of coenzyme is 10% to 30%, and the mass fraction of the electron mediator is 10% to 30%. In this case, the reaction sensitivity of the second analyte enzyme layer against β-BHB can be improved, and meanwhile, a macromolecular electron mediator is disposed in the enzyme layer, β-hydroxybutyrate dehydrogenase can be covalently bound to the electron mediator, resulting in further improving the stability.

In addition, in the multi-analyte monitoring sensor according to the present disclosure, optionally, the sensor further includes a reference electrode and a counter electrode, wherein the first working electrode and the second working electrode are respectively positioned on opposite sides of the substrate, and the reference electrode and the counter electrode are respectively positioned on both sides of the substrate. In this case, the sensor of four-electrode and dual-channel detection can be formed, and the four electrodes disposed in pairs on both sides of the substrate can improve the utilization of the substrate, while further reducing the interference of the electric field on the sensor system.

According to the present disclosure, a multi-analyte monitoring sensor capable of continuously monitoring glucose and ketone simultaneously, with simple manufacturing processes, high sensitivity, and stability can be provided.

### BRIEF DESCRIPTION OF THE DRAWINGS

The present disclosure will now be explained in further details by reference to the examples in the accompanying drawings only, in which:
Fig.1 is a schematic diagram showing the multi-analyte monitoring sensor according to an example of the present disclosure.
Fig.2A is a schematic diagram showing the front side of the multi-analyte monitoring sensor according to an example of the present disclosure.
Fig.2B is a schematic diagram showing the back side of the multi-analyte monitoring sensor according to an example of the present disclosure.
Fig.3 is a schematic diagram showing the polymer membrane layer according to an example of the present disclosure.
Fig.4 is a curve graph showing the response current-test concentration of Embodiment 1 of the blood glucose + blood ketone sensor according to the present disclosure.
Fig.5 is a photograph showing the monitoring probe of Embodiment 2 of the blood glucose + blood ketone sensor according to the present disclosure.
Fig.6 is a curve graph showing the response current-test time of Embodiment 2 of the blood glucose + blood ketone sensor according to the present disclosure.
Fig.7 is a curve graph showing the response current-test concentration of Embodiment 2 of the blood glucose + blood ketone sensor according to the present disclosure.
Fig.8 is a graph showing the glucose/sodium β-hydroxybutyrate response current-time curve of Embodiment 2 of the blood glucose + blood ketone sensor related to the present disclosure.
Fig.9 is a curve graph showing the response current-test concentration of Embodiment 3 of the blood glucose + blood ketone sensor according to the present disclosure.
Fig.10 is a photograph showing the monitoring probe of Comparative Example 1 of the blood glucose + blood ketone sensor according to the present disclosure.
Fig.11 is a curve graph showing the response current-test concentration of Comparative Example 1 of the blood glucose + blood ketone sensor according to the present disclosure.
Fig.12 is a photograph showing the monitoring probe of Comparative Example 2 of the blood glucose + blood ketone sensor according to the present disclosure.
Fig.13 is a curve graph showing the response current-test concentration of Comparative Example 2 of the blood glucose + blood ketone sensor according to the present disclosure.
Fig.14 is a photograph showing the monitoring probe of Comparative Example 3 of the blood glucose + blood ketone sensor according to the present disclosure.
Fig.15 is a photograph showing another view of the monitoring probe of Comparative Example 3 of the blood glucose + blood ketone sensor according to the present disclosure.
Fig.16 is a photograph showing the monitoring probe of the sensor in Embodiment 1, Comparative Examples 1-2 of the blood ketone sensor according to the present disclosure.
Fig.17 is a photograph showing the monitoring probe coated with polymer membrane layer in Embodiment 1, Comparative Examples 1-2 of the blood ketone sensor according to the present disclosure.
Fig.18 is a graph showing the response current-test concentration of the linear test of Embodiment 1 of the blood ketone sensor according to the present disclosure.
Fig.19 is a curve graph showing the response current-test concentration of the linear test of Embodiment 1 of the blood ketone sensor according to the present disclosure.
Fig.20 is a curve graph showing the response current-test concentration of the stability test of Embodiment 1 of the blood ketone sensor according to the present disclosure.
Fig.21 is a curve graph showing the response current-test concentration of the stability test of Comparative Example 1 of the blood ketone sensor according to the present disclosure.
Fig.22 is a curve graph showing the response current-test concentration of the stability test of Comparative Example 2 of the blood ketone sensor according to the present disclosure.
Fig.23 is a graph showing the response current-test concentration of the linear test of Comparative Example 3 of the blood ketone sensor according to the present disclosure.
Fig.24 is a photograph showing the working electrode of Comparative Example 4 of the blood ketone sensor according to the present disclosure.
Fig.25 is a photograph showing the monitoring probe of the sensor of Comparative Example 4 of the blood ketone sensor according to the present disclosure.
Fig.26 is a curve graph showing the response current-test time of the sensor of Comparative Example 4 of the blood ketone sensor according to the present disclosure.
Fig.27 is a photograph showing the working electrode of the sensor of Comparative Example 5 of the blood ketone sensor according to the present disclosure.
Fig.28 is a graph showing the response current-test concentration of the linear test of Comparative Example 5 of the blood ketone sensor according to the present disclosure.

### Description of reference numerals of the accompanying drawings:

10... Monitoring probe, 11... Substrate, 12...First working electrode, 121...First analyte enzyme layer, 13...Second working electrode, 131...Second analyte enzyme layer, 14...Semi-permeable membrane, 141...First membrane layer, 142...Transition layer, 143...Second membrane layer, 15...Reference electrode, 16...Counter electrode

### DETAILED DESCRIPTION

Hereinafter, preferred embodiments of the present disclosure will be described in detail with reference to the accompanying drawings. In the following descriptions, identical components are assigned the same symbols, and redundant explanations are omitted. In addition, the accompany drawings are merely schematic diagrams, and the proportions of the sizes or shapes of the components relative to each other may differ from actual implementations.

It should be noted that the terms "include" and "have", and any variations thereof in the present disclosure, such as a process, method, system, product, or equipment that includes or has a series of steps or elements are not necessarily limited to those explicitly recited steps or elements, but may include or have other steps or elements not explicitly recited or inherent to such process, method, product, or equipment.

In addition, the subheadings and the like mentioned in the following description of the present disclosure are not intended to limit the content or scope of the present disclosure, they are merely provided as reading cues. Such subheadings should not be construed as segmenting the content of the text, nor should the content under the subheadings be limited only to the scope of the subheadings.

One aspect of the present disclosure relates to a multi-analyte monitoring sensor, another aspect relates to a semi-permeable membrane for the multi-analyte monitoring sensor, and a third aspect relates to a blood ketone sensor.

In the present disclosure, the multi-analyte monitoring sensor may also be referred to "multi-analyte sensor," "continuous blood glucose and blood ketone monitoring sensor," "blood glucose and blood ketone sensor," "blood glucose + blood ketone sensor," "physiological parameter sensor," or simply as "sensor," "sensing probe," "sensing tip," "implantation probe," etc.

The sensor according to the present disclosure may be used to monitor physiological parameters of a host. The physiological parameters may include glucose, urea, uric acid, ketone bodies, and a series of amino acid compounds within the host's body.

In the present disclosure, the sensor may also be used to detect analytes within the host's body. The analytes may be chemical substances in bodily fluids. For example, the analytes may be one or more of glucose, acetylcholine, amylase, bilirubin, cholesterol, chorionic gonadotropin, creatine kinase, creatine, creatinine, DNA, fructosamine, glucose, glutamine, growth hormone, hormone, ketone bodies, lactate, oxygen, peroxide, prostate-specific antigen, prothrombin, RNA, thyroid-stimulating hormone, or troponin. Additionally, the analytes may also be medications in bodily fluids. For example, the analytes may be digitoxin, digoxin, theophylline, warfarin, or antibiotics (such as gentamicin, vancomycin, etc.).

Hereinafter, the multi-analyte monitoring sensor relates to the first aspect of the present disclosure will be described combined with the accompanying drawings.

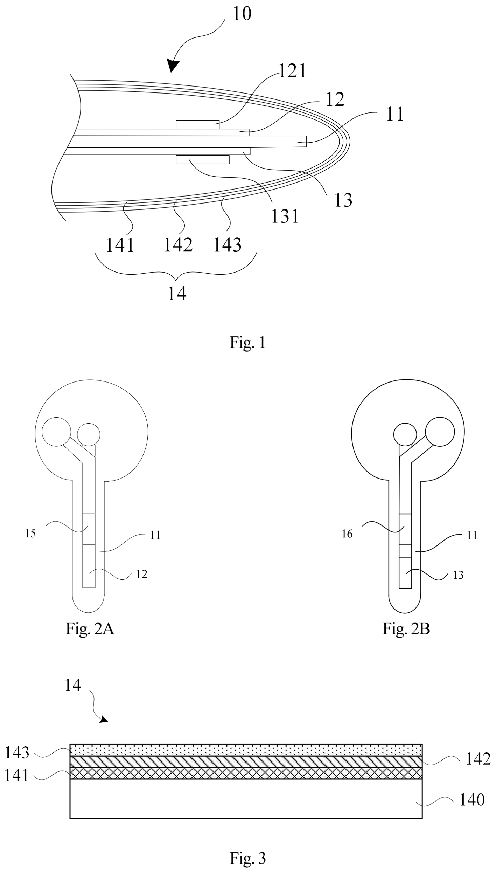

Fig.1 is a schematic diagram showing the multi-analyte monitoring sensor according to an example of the present disclosure.

In the present embodiment, sensor 10 may be used to obtain physiological parameter information from a host. In some examples, sensor 10 may be applied to a host, and the host may obtain his/her own physiological parameter information through monitoring device 1 attached to himself/herself. In some examples, sensor 10 may be mounted on the host's body surface, such as the arm, back, abdomen, waist, leg, etc., where sensor 10 may be partially implanted to monitor physiological parameters. In some examples, sensor 10 may be used for implantation under the host's skin to obtain sensing signals (e.g., electrical current signals). In some examples, when sensor 10 is applied to the host, it may come into contact with the host's tissue fluid or blood to measure the level of analytes in the tissue fluid or blood.

**In** some examples, sensor 10 includes a substrate 11 and a working electrode disposed on the substrate. In some examples, the number of working electrode may be plural, and each working electrode may be used to detect different analyte. Thereby, sensor 10 is capable of monitoring a plurality of analytes.

In some examples, the working electrode may include a first working electrode 12 and a second working electrode 13. The first working electrode 12 may be used to detect a first analyte, and the second working electrode 13 may be used to detect a second analyte. Thereby, two different analytes can be monitored by sensor 10.

In some examples, the first analyte may be any one of glucose, urea, uric acid, ketone bodies, creatinine, ethanol, and lactic acid. In some examples, the second analyte may also be any one of glucose, urea, uric acid, ketone bodies, creatinine, ethanol, and lactic acid. Wherein, ketone bodies (which may be simply referred to as ketones) are collectively referred to as the transition products of fatty acid oxidation and decomposition in the liver, including acetoacetic acid, β-hydroxybutyric acid, and acetone. In the present disclosure, the content of ketone bodies may be monitored by detecting any one of acetoacetic acid, β-hydroxybutyric acid, and acetone. Preferably, the β-hydroxybutyric acid in the host body may be monitored to obtain parameters related to the ketone bodies level.

In some examples, the first analyte may be glucose. Thereby, the glucose concentration in the body can be monitored. In some examples, the second analyte may be ketone bodies. Thereby, the ketone bodies content in the body can be monitored. It should be noted that, as mentioned above, ketone bodies are collectively referred to as the transition products of fatty acid oxidation and decomposition in the liver, including acetoacetic acid, β-hydroxybutyric acid. Thus, the second analyte may be any one of acetoacetic acid, β-hydroxybutyric acid, and acetone. Preferably, the second analyte may be β-hydroxybutyric acid.

Hereinafter, taking glucose as the first analyte and ketone bodies as the second analyte as an example, sensor 10 according to the present disclosure will be described in detail.

In some examples, sensor 10 may include an enzyme sensing layer disposed on the working electrode. In the example where the working electrode includes a first working electrode 12 and a second working electrode 13, the enzyme sensing layer may include a first analyte enzyme layer 121 and a second analyte enzyme layer 131. The first analyte enzyme layer 121 may be disposed on the first working electrode 12, and the second analyte enzyme layer 131 may be disposed on the second working electrode 13. In this case, the enzyme layers for two different analytes are disposed on the two working electrodes of sensor 10, thereby the concentration levels of the two different analytes in the tissue fluids can be detected respectively.

In some examples, sensor 10 may include a polymer membrane layer 14. The polymer membrane layer 14 may be configured to be permeable to the first analyte and the second analyte and cover the first analyte enzyme layer 121 and the second analyte enzyme layer 131. In this case, the polymer membrane layer 14 can restrict the permeation of the two analytes while facilitating the improvement of stability of the sensor. Compared to the scheme of separately disposing semi-permeable membranes permeable to the first analyte and the second analyte on the first analyte enzyme layer 121 and the second analyte enzyme layer 131, the polymer membrane 14 in the present disclosure can simultaneously permeate the first analyte and the second analyte, thereby simplifying the manufacturing process of sensor 10 while maintaining high sensitivity and accuracy. In some examples, the polymer membrane layer 14 can simultaneously permeate glucose and ketones. In this case, the first analyte enzyme layer 121 and the second analyte enzyme layer 131 are covered with the polymer membrane layer 14, the permeation of the two analytes can be restricted while facilitating the improvement of stability of the sensor. In addition, due to the small size of the biosensor 10, high precision is required for coating the surface of sensor 10. Usually, the dipping and pulling method is used to cover the surface of sensor 10 with a membrane solution to form a membrane layer coating sensor 10. Compared to the scheme of separately disposing semi-permeable membranes permeable to glucose and ketones on the first analyte enzyme layer 121 and the second analyte enzyme layer 131, the polymer membrane 14 in the present disclosure can simultaneously permeate both glucose and ketones, thereby simplifying the manufacturing process of sensor 10 while maintaining high sensitivity and accuracy. Thus, sensor 10 with a simple manufacturing process capable of continuous monitoring of glucose and ketones can be provided.

In some examples, the permeability coefficient of the polymer membrane layer 14 for glucose may be 1 to 1000. Preferably, the permeability coefficient of the polymer membrane layer 14 for glucose may be 300 to 800. For example, the permeability coefficient of the polymer membrane layer 14 for glucose may be 300, 400, 500, 600, 700, or 800. In some examples, the permeability coefficient of the polymer membrane layer 14 for ketones may be 1 to 1000. Preferably, the permeability coefficient of the polymer membrane layer 14 for ketones may be 200 to 600. For example, the permeability coefficient of the polymer membrane layer 14 for ketones may be 200, 300, 400, 500, or 600. Wherein, the permeability coefficient refers to the concentration difference inside and outside the polymer membrane layer. For example, if the permeability coefficient of the polymer membrane layer 14 for glucose is 500, then the concentration of glucose outside the polymer membrane layer is 500 times the concentration of glucose inside the polymer membrane layer.

In some examples, the polymer membrane layer 14 may have different permeability coefficients by adjusting the thickness ratio of the polymer membrane layer 14. In this case, the permeability coefficient of the polymer membrane layer 14 is adjusted, the amount of permeating analyte can be controlled, making the current generated by the working electrode more accurate, resulting in enabling sensor 10 to be more sensitive.

Fig.2A is a schematic diagram showing the front side of the multi-analyte monitoring sensor according to an example of the present disclosure. Fig.2B is a schematic diagram showing the back side of the multi-analyte monitoring sensor according to an example of the present disclosure.

In some examples, sensor 10 may be used in conjunction with an electronic system, sensor 10 may be combined with the electronic system to form the sensing assembly. Specifically, sensor 10 may be electrically connected to the electronic system, where sensor 10 can partially be implanted under the skin of a host to acquire sensing signals indicating the level of an analyte and then transmit the sensing signals to the electronic system; The electronic system may process and/or retransmit the sensing signals. In this case, the concentration of the analyte in the body can be accurately acquired.

In some examples, sensor 10 may further include a reference electrode 15 and a counter electrode 16 (Referred to Fig.2A and Fig.2B). In this case, a four-electrode with dual-path detection sensor can be formed, where the first working electrode 12, the reference electrode 15, and the counter electrode 16 may form a set of three-electrode circuits, and similarly, the second working electrode 13 may also form a set of three-electrode circuits with the reference electrode 15 and the counter electrode 16.

In some examples, the four-electrode with dual-path detection sensor may have a first working electrode 12, a second working electrode 13, a reference electrode 15, and a counter electrode 16. The first working electrode 12 forms a first circuit with the reference electrode 15 and the counter electrode 16, while the second working electrode 13 forms a second circuit with the reference electrode 15 and the counter electrode 16. In this case, the number of electrodes in the sensor can be reduced, thereby the length of the substrate can be reduced, resulting in reducing the implantation depth of sensor 10. Since greater implantation depth leads to more obvious pain, thus, the implantation depth of sensor 10 can be reduced to improve the comfort of wearing.

In some examples, the first working electrode 12 and the second working electrode 13 may be respectively positioned on both sides of the substrate 11, and similarly, the reference electrode 15 and the counter electrode 16 are respectively positioned on both sides of the substrate 11. In this case, the four electrodes are respectively disposed in pairs on both sides of the substrate can improve the utilization of the substrate while optimizing the voltage drop of the sensor electrodes, the errors can be reduced, and the interference of the electric field on the sensor system can be further reduced. In some examples, since the comfort of wearing is related to the implantation depth, a longer substrate results in a greater implantation depth, potentially increasing discomfort. Hence, the four electrodes are respectively disposed in pairs on both sides of the substrate can improve the utilization of the substrate, four electrodes are disposed within a limited substrate space, the length of the substrate can be reduced, thereby improving the comfort of wearing.

In some examples, reference electrode 15 may form a known and fixed potential difference with the tissue fluid or blood. In this case, the potential difference between the working electrode and the tissue fluid or blood may be measured through the potential difference formed by reference electrode 15 and the working electrode. Thus, the voltage generated by the working electrode can be obtained more accurately, and the electronic system can automatically adjust and maintain the stability of the voltage at the working electrode based on the preset voltage value, so that the measured current signal can more accurately reflect the analyte concentration level information in the tissue fluid or blood. In some examples, the number of reference electrode 15 may be one or more, such as two. In this case, the number of the reference electrode can be adjusted according to the number of working electrode or the current generated by the working electrode, thereby accurate current signals can be obtained, resulting in improving the sensitivity of the sensor.

In some examples, sensor 10 may include an implantable portion and a connecting portion. The implantable portion may be placed under the skin of a host and connected to the electronic system through the connecting portion. In this case, the implantable portion is placed under the skin of the host and electrically connecting the implantable portion to the electronic system through the connecting portion, thereby facilitating the transmission of the sensing signal obtained by the implantable portion to the electronic system. In some examples, the implantable portion may be rigid. Thus, the implantable portion to be placed under the skin of the host can be facilitated. In other examples, the implantable portion may also be flexible, in this case, the foreign body sensation of the host can be reduced.

In some examples, as mentioned above, sensor 10 may include first analyte enzyme layer 121. In this case, the first analyte capable of reacting in first analyte enzyme layer 121, thereby generating currents, and a concentration signal of the first analyte can be obtained

In some examples, first analyte enzyme layer 121 may include glucose oxidase. Thus, the glucose capable of reacting in the first analyte enzyme layer, resulting in generating a concentration signal of glucose. In some examples, the content (mass fraction) of glucose oxidase in first analyte enzyme layer 121 may range from 40% to 70%. For example, the content (mass fraction) of glucose oxidase may be 40%, 41%, 42%, 43%, 44%, 45%, 46%, 47%, 48%, 49%, 50%, 51%, 52%, 53%, 54%, 55%, 56%, 57%, 58%, 59%, 60%, 61%, 61%, 62%, 63%, 64%, 65%, 66%, 67%, 68%, 69%, or 70%. Thus, the reaction of glucose can be facilitated.

In some examples, first analyte enzyme layer 121 may include an electron mediator. In some examples, the electron mediator may be a redox polymer. In some examples, the redox polymer may be a metal-based redox polymer. In some examples, the metal-based redox polymer may be selected from at least one of poly(vinylferrocene), quaternized poly(4-vinylpyridine) ferricyanide, quaternized poly(1-vinylimidazole) ferricyanide, quaternized poly(4-vinylpyridine) ferrocyanide, quaternized poly(1-vinylimidazole) ferrocyanide, osmium 2,2'-bipyridine complex coordinated to poly(1-vinylimidazole), osmium 2,2'-bipyridine complex coordinated to poly(4-vinylpyridine), ruthenium 2,2'-bipyridine complex coordinated to poly(1-vinylimidazole), ruthenium 2,2'-bipyridine complex coordinated to poly(4-vinylpyridine), cobalt 2,2'-bipyridine complex coordinated to poly(1-vinylimidazole), or cobalt 2,2'-bipyridine complex coordinated to poly(4-vinylpyridine). Thus, the metal-based redox polymer can participate in redox reactions through covalent bonds, coordination bonds, or ionic bonds.

In some examples, the content (mass fraction) of the electron mediator in first analyte enzyme layer 121 may range from 10% to 40%. For example, the content (mass fraction) of the electron mediator may be 10%, 11%, 12%, 13%, 14%, 15%, 16%, 17%, 18%, 19%, 20%, 21%, 22%, 25%, 26%, 27%, 28%, 29%, 30%, 31%, 32%, 33%, 34%, 35%, 36%, 37%, 38%, 39%, or 40%. Thus, the reaction of the first analyte in the first analyte enzyme layer can be facilitated.

In some examples, first analyte enzyme layer 121 may include a cross-linking agent. In some examples, the cross-linking agent may be PEGDGE. In some examples, the cross-linking agent may be PEGDGE400. In some examples, the content (mass fraction) of the cross-linking agent in first analyte enzyme layer 121 may range from 0% to 20%. For example, the content (mass fraction) of the cross-linking agent may be 0%, 1%, 2%, 3%, 4%, 5%, 6%, 7%, 8%, 9%, 10%, 11%, 12%, 13%, 14%, 15%, 16%, 17%, 18%, 19%, or 20%. Thus, longtime monitoring of the sensor can be facilitated.

In some examples, the area of first analyte enzyme layer 121 may be greater than the area of second analyte enzyme layer 131. Since the amount of glucose and ketone permeable through polymer membrane 14 is different, and usually, the amount of glucose is less than the amount of ketone. In this case, the area of first analyte enzyme layer 121 is configured to be greater than the area of second analyte enzyme layer 131, the contactable area between first analyte enzyme layer 121 and glucose can be thereby increased, resulting in reducing the current difference between the two working electrodes as much as possible, ensuring that the current levels on both working electrodes are at the same level, reducing the interference of the electric field on sensor 10 system, further improving the measurement accuracy of sensor 10.

In some examples, the area ratio of first analyte enzyme layer 121 to second analyte enzyme layer 131 may be greater than 1:1. Thus, the improvement of sensitivity of sensor 10 can be facilitated. In some examples, the area ratio of first analyte enzyme layer 121 to second analyte enzyme layer 131 may be below 1.5:1. Thereby, the accuracy of sensor 10 can be improved. In some examples, the area ratio of first analyte enzyme layer 121 to second analyte enzyme layer 131 may be 1:1 to 1.5:1. Thus, the reaction sensitivity of the first analyte can be improved, resulting in accurately reflecting the concentration level of the analyte in the tissue fluid. In some examples, the area ratio of second analyte enzyme layer 131 to first analyte enzyme layer 121 may be 1:1.5, 1:1.4, 1:1.3, 1:1.2, 1:1.1. Thus, the concentration level of the analyte in the tissue fluid can be more accurately reflected.

In some examples, first analyte enzyme layer 121 and/or second analyte enzyme layer 131 may be in a linear shape. Thus, the utilization rate of the substrate can be improved, the sensitivity of the sensor can be improved at the same time. As limited by the existing processing level, due to the small size of the sensor, when the working electrode of the sensor is coated with a very small analyte enzyme layer, it is difficult to directly coat the electrode surface with a uniform linear enzyme layer with the existing processing accuracy. In some examples of the present disclosure, a solution containing the enzyme layer may be drop-coated onto the working electrode, and the dot-shaped solution droplets may spread and connected to form a continuous linear solution. In such case, compared to the scheme of multi-point discrete arrangement, a larger area of enzyme layer can be arranged on the limited area of the working electrode, thereby improving the sensitivity of sensor 10. In some examples, the two adjacent droplets can be intersected during the drop-coating process, thereby a linear analyte enzyme layer can be formed on the working electrode in a manner of multi-point connection. In this case, the processing difficulty of sensor 10 can be reduced by connecting multiple points to a line, thereby the linear analyte enzyme layer can be formed, resulting in improving the utilization rate of the substrate.

In the present disclosure, "first analyte enzyme layer 121 and/or second analyte enzyme layer 131 being in a linear shape" includes at least three embodiments: first analyte enzyme layer 121 being in the linear shape, second analyte enzyme layer 131 being in the linear shape, and both first analyte enzyme layers 121 and second analyte enzyme layer 131 being in a linear shape. In the present disclosure, other expressions using "A and/or B" have the same meaning as mentioned above. In this case, the area of the analyte enzyme layer is adjusted, thereby the improvement of sensitivity and accuracy of sensor 10 can be facilitated when monitoring two analytes.

Fig.3 is a schematic diagram showing polymer membrane layer 14 according to an example of the present disclosure. In Fig.3, the schematic representation of the analyte enzyme layer is omitted.

In some examples, first working electrode 12 may have base layer 140. Thus, the stability of sensor 10 can be improved. In some examples, base layer 140 is of electrical conductivity, enabling it to conduct the current signal generated by the analyte reaction. In some examples, base layer 140 may be made from at least one material selected from gold, glassy carbon, graphite, silver, silver chloride, palladium, titanium, and iridium. In this case, base layer 140 can have good electrical conductivity, and can inhibit electrochemical reactions in base layer 140, thus, the stability of base layer 140 can be improved.

In some examples, polymer membrane layer 14 may be coated on base layer 140 of the working electrode (Referred to Fig.3). Thus, the membrane layer fabrication process of sensor 10 can be simplified, and at the same time, the biocompatibility of sensor 10 can be improved, resulting in facilitating the longtime use of sensor 10.

In some examples, polymer membrane layer 14 may include vinyl pyridine-based polymers. Thus, the formation of polymer membrane layer 14 with limited permeability to glucose and ketones can be facilitated.

In some examples, the mass fraction of the vinyl pyridine-based polymer in polymer membrane layer 14 ranges from 80% to 100%. Thus, the formation of biocompatible polymer membrane layer 14 with limited permeability to glucose and ketones can be facilitated, the amount of glucose and ketones can be controlled within the linear range of the sensor through the polymer membrane, resulting in improving the sensitivity and accuracy of the sensor. In some examples, the mass fraction of the vinyl pyridine-based polymer in polymer membrane layer 14 may be 80%, 81%, 82%, 83%, 84%, 85%, 86%, 87%, 88%, 89%, 90%, 91%, 92%, 93%, 94%, 95%, 96%, 97%, 98%, 99%, or 100%. Thus, the formation of stable polymer membrane layer 14 can be facilitated, at the same time, the limited permeability of polymer membrane layer 14 to glucose and ketones can be facilitated.

In some examples, polymer membrane layer 14 may further include a crosslinking agent and a modifier. The crosslinking agent may be polyethylene glycol diglycidyl ether, and the modifier may be polydimethylsiloxane modifier. Thus, the stability of polymer membrane layer 14 can be improved, thereby improving the performance of sensor 10

In some examples, polymer membrane layer 14 may include first membrane layer 141. First membrane layer 141 may be disposed on the enzyme sensing layer. Thus, the permeation of analytes in the tissue fluid can be limited, at the same time, the permeation of other interfering substances can be prevented, the reaction efficiency of the enzyme sensing layer can be improved, and thereby improving the sensitivity and stability of the sensor.

In some examples, polymer membrane layer 14 may include second membrane layer 143. Second membrane layer 143 may have biocompatibility. Thus, the occurrence of inflammatory phenomena caused by the implantation of sensor 10 in the body can be reduced. In some examples, second membrane layer 143 may be disposed on first membrane layer 141. In this case, polymer membrane layer 14 with both limiting permeability and biocompatibility can be formed, thereby facilitating the improvement of sensitivity and stability of sensor 10.

In some examples, polymer membrane layer 14 may further include transition layer 142. Transition layer 142 may be disposed between first membrane layer 141 and second membrane layer 143. Thus, stable polymer membrane layer 14 can be formed. In other words, in some examples, polymer membrane layer 14 may successively include first membrane layer 141 disposed on the enzyme sensing layer, transition layer 142 formed on first membrane layer 141, and second membrane layer 143 with biocompatibility formed on transition layer 142. Thus, the adhesive stability of polymer membrane layer 14 can be improved, thereby facilitating the improvement of stability of sensor 10.

In some examples, first membrane layer 141 is formed by a first type of polymer. In some examples, the first type of polymer may include water-swellable homopolymers. Thus, the formation of the first membrane layer can be facilitated. In some examples, the water-swellable homopolymers may be selected from one of polystyrene, polyurethane, poly(ethyl acrylate), poly(propyl acrylate), poly(2-vinylpyridine), poly(4-vinylpyridine), poly(hydroxyethyl methacrylate), and poly(hydroxyethyl acrylate). Thus, the membrane layer with diffusion control properties can be formed, resulting in a fixed concentration ratio between the analyte concentrations on both sides of the polymer membrane layer, thereby expanding the linear range of biosensor response.

In some examples, second membrane layer 143 is formed by a second type of polymer. In some examples, the second type of polymer may include water-soluble polymers. In some examples, the water-soluble polymers may be selected from one of polyvinyl pyrrolidone, polyvinyl alcohol, chitosan, carboxymethyl chitosan, chitosan salts, alginic acid, alginate salts, hyaluronic acid, hyaluronic acid salts, cellulose ethers, cellulose esters, polyvinyl pyrrolidone, polyacrylamide, polyacrylic acid, polyvinyl alcohol, sodium polystyrene sulfonate, polyethylene glycol, and polyethylene glycol-polypropylene glycol copolymers. Thus, the biocompatibility of the biosensor can be improved.

In some examples, transition layer 142 is formed by a third type of polymer. The third type of polymer is a copolymer formed by a first monomer that is the same as or similar to the first type of polymer and a second monomer that is the same as or similar to the second type of polymer. Thus, the adhesive stability and mechanical strength of polymer membrane layer 14 can be improved, thereby improving the stability of sensor 10. Thus, a transition layer with enhanced adhesion to first membrane layer 141 and second membrane layer 143 can be formed, thereby facilitating the formation of polymer membrane layer 14, resulting in facilitating the expansion of the response linear range of sensor 10 and improving the biocompatibility of sensor 10.

In some examples, first membrane layer 141 may include a vinyl pyridine-based polymer. Thus, the formation of a membrane layer with limited permeability to two analytes can be formed.

In some examples, transition layer 142 may include a copolymer of vinyl pyridine and styrene. Thus, the formation of a uniform and stable polymer membrane layer 14 can be formed, thereby improving the stability of the polymer membrane layer.

In some examples, second membrane layer 143 may include a copolymer of vinyl pyridine and styrene. Thus, the improvement of the membrane-forming properties of polymer membrane layer 14 can be facilitated, thereby improving the sensitivity of sensor 10.

In some examples, second analyte enzyme layer 131 may include hydroxybutyrate dehydrogenase. In this case, the hydroxybutyrate in the tissue fluid can react under the effect of hydroxybutyrate dehydrogenase, thereby the concentration level of hydroxybutyrate can be obtained through the signal generated by the reaction, and further mapping the ketone concentration in the tissue fluid based on the concentration level of hydroxybutyrate. In some examples, the content of hydroxybutyrate dehydrogenase in second analyte enzyme layer 131 may range from 10% to 20%. For example, the content of hydroxybutyrate dehydrogenase may be 10%, 11%, 12%, 13%, 14%, 15%, 16%, 17%, 18%, 19%, or 20%. Thus, the redox reaction of ketones in second analyte enzyme layer 131 can be facilitated.

In some examples, second analyte enzyme layer 131 may include diaphorase. In this case, the electrons generated by the β-BHB reaction can be transferred through diaphorase. In some examples, the content of diaphorase in second analyte enzyme layer 131 may range from 5% to 20%. For example, the content of diaphorase may be 5%, 6%, 7%, 8%, 9%, 10%, 11%, 12%, 13%, 14%, 15%, 16%, 17%, 18%, 19%, or 20%. Thus, the reaction in second analyte enzyme layer 131 can be facilitated, at the same time, the improvement the electron transfer efficiency in the working electrode can be improved.

In some examples, second analyte enzyme layer 131 may include coenzyme. Thus, the oxidation of hydroxybutyrate dehydrogenase can be facilitated, thereby transferring the electrons generated by the β-BHB reaction. In some examples, the content of coenzyme in second analyte enzyme layer 131 may range from 10% to 30%. For instance, the content of coenzyme may be 10%, 12%, 14%, 15%, 16%, 18%, 19%, 20%, 21%, 22%, 25%, 26%, 27%, 28%, 29%, or 30%. Thus, the redox reaction between hydroxybutyrate dehydrogenase and β-BHB cam be facilitated.

In some examples, second analyte enzyme layer 131 may include an electron mediator. In some examples, the electron mediator may be a redox polymer. In some examples, the redox polymer may be a metal-based redox polymer. In some examples, the metal-based redox polymer may be selected from at least one of poly(vinylferrocene), quaternized poly(4-vinylpyridine) ferricyanide, quaternized poly(1-vinylimidazole) ferricyanide, quaternized poly(4-vinylpyridine) ferrocyanide, quaternized poly(1-vinylimidazole) ferrocyanide, osmium 2,2'-bipyridine complex coordinated to poly(1-vinylimidazole), osmium 2,2'-bipyridine complex coordinated to poly(4-vinylpyridine), ruthenium 2,2'-bipyridine complex coordinated to poly(1-vinylimidazole), ruthenium 2,2'-bipyridine complex coordinated to poly(4-vinylpyridine), cobalt 2,2'-bipyridine complex coordinated to poly(1-vinylimidazole), or cobalt 2,2'-bipyridine complex coordinated to poly(4-vinylpyridine). Thus, the metal-based redox polymer can participate in the redox reaction through covalent bonds, coordination bonds, or ionic bonds, thereby facilitating the transfer of electrons to the working electrode, resulting in generating a current signal corresponding to the concentration of the second analyte. In some examples, the content of the electron mediator in second analyte enzyme layer 131 may range from 10% to 30%. For example, the content may be 10%, 12%, 14%, 15%, 16%, 18%, 19%, 20%, 21%, 22%, 25%, 26%, 27%, 28%, 29%, or 30%. In this case, the reaction sensitivity of second analyte enzyme layer 131 to β-BHB can be improved. Meanwhile, a large-molecule electron mediator is disposed in the enzyme layer, enabling hydroxybutyrate dehydrogenase covalently bound to the electron mediator, thereby further improving the stability.

According to the first aspect of the present disclosure, a multi-analyte monitoring sensor capable of continuously monitoring both glucose and ketones with a simple fabrication process, high sensitivity and stability.

The second aspect of the present disclosure relates to a semi-permeable membrane for the multi-analyte monitoring sensor. The semi-permeable membrane according to the second aspect of the present disclosure is in accordance with polymer membrane layer 14 in sensor 10 described in the first aspect of the present disclosure, as for the structure of the polymer membrane layer 14, the settings of its various components and parameters, as well as the preparation method may refer to the descriptions mentioned above, further elaboration is omitted here.

In the second aspect of the present disclosure, a semi-permeable membrane with high stability and capable of having limited permeability to both glucose and ketones can be provided. The semi-permeable membrane is coated on the electrodes of multi-analyte sensor 10 can facilitate the improvement of sensitivity of sensor 10.

The third aspect of the present disclosure relates to a blood ketone sensor. The blood ketone sensor according to the third aspect of the present disclosure is basically consistent with sensor 10 described in the first aspect of the present disclosure, with the exception that the first working electrode and the first analyte enzyme layer disposed on the first working electrode are removed, retaining the second working electrode and the second analyte enzyme layer (i.e., the enzyme layer for detecting ketones) which use for ketones analysis, other parts remain the same as sensor 10 described in the first aspect, and further elaboration is omitted here. In some examples, in the blood ketone sensor, second working electrode 13 and reference electrode 15 can be disposed on the same side of substrate 11, while counter electrode 16 may be disposed on the other side of substrate 11. Thus, the utilization of the substrate can be improved. Through the blood ketone sensor according to the third aspect of the present disclosure, the ketone level in the host's body can be measured.

Hereinafter, the multi-analyte monitoring sensor and blood ketone sensor provided in the present disclosure will be described in detail with reference to embodiments and Comparative Examples, but they should not be understood as limiting the scope of protection of the present disclosure.

### [Blood Glucose + Blood Ketone Sensor]

### Embodiment 1

Firstly, preparing a monitoring probe with dual working electrodes in front and rear, respectively referred to as the first working electrode and the second working electrode.

Secondly, preparing the reagent compositions (osmium-coordinated metal polymer, β-hydroxybutyrate dehydrogenase, diaphorase, NAD+, stabilizer, cross-linker) of ketone-sensitive layer according to Table 1. Dissolving each substance in the buffered solvent of HEPES buffer solution according to the concentration parameters of the table, a β-hydroxybutyrate-sensitive layer solution is obtained.

Subsequently, obtaining a glucose-sensitive layer solution by formulating osmium-coordinated metal polymer with concentration of 10 mg/mL, glucose oxidase (GOD) with concentration of 12.5 mg/mL, and cross-linker PEGDGE with concentration of 2.5 mg/mL.

The glucose-sensitive layer solution is deposited on the first working electrode, the β-hydroxybutyrate-sensitive layer solution is deposited on the second working electrode, both forming a linear pattern. Wherein the area of the glucose sensing layer is approximately 0.27 mm2, and the area of the ketone body sensing layer is approximately 0.27 mm2; that is the area ratio of the blood ketone to blood glucose sensing layers is 1:1; A dual-component responsive sensing layer electrode incorporating both glucose and ketone bodies is obtained after curing.

**Table 1. Composition of ketone body-sensitive layer reagent**

| Substance | Concentration (mg/mL) |
|---|---|
| Metal polymer | 10 |
| β-Hydroxybutyrate dehydrogenase | 6 |
| Diaphorase | 6 |
| NAD⁺ | 6 |
| Proserum | 6 |
| PEGDGE | 5 |

And then, ethanol is prepared as the solvent, and polyvinyl pyridine and PEGDGE are formulated according to Table 2 to obtain a diffusion-limiting membrane solution. The diffusion-limiting membrane solution is coated onto the electrode deposited with the sensitive layer reagent to form a dual-component monitoring working electrode incorporating a membrane layer of approximately 25 micrometers.

**Table 2. Composition of diffusion-limiting membrane solution**

| Substance | Concentration (mg/mL) |
|---|---|
| Polyvinyl pyridine | 100 |
| PDMS | 4 |
| PEGDGE | 35 |

Preparing an 80% ethanol-water solution, a solution of poly(4-vinylpyridine-co-styrene) and PEGDGE is formulated at the corresponding concentrations according to Table 3 to obtain a compatible membrane solution; the compatible membrane solution is coated onto the diffusion-limiting membrane of the working electrode to form a working electrode incorporating a compatible membrane layer of approximately 15 micrometers.

**Table 3. Composition of compatible membrane solution**

| Substance | Concentration (mg/mL) |
|---|---|
| Poly4 - vinyl pyridine - b - polystyrene | 100 |
| PEGDGE | 15 |

Preparing an 80% ethanol-water solution, a solution of (poly(4-vinylpyridine-g-polyethylene glycol)-co-styrene) and PEGDGE is formulated at corresponding concentrations according to Table 4 to obtain a biocompatible membrane solution; The biocompatible membrane solution is coated onto the compatible membrane of the working electrode to form a working electrode incorporating a biocompatible membrane layer of approximately 15 micrometers, with a total membrane thickness of approximately 56 micrometers.

**Table 4. Composition of biocompatible membrane solution**

| Substance | Concentration (mg/mL) |
|---|---|
| (Poly4 - vinylpyridine -g- polyethylene glycol) - co - polystyrene | 100 |
| PEGDGE | 15 |

The sensor of Embodiment 1 is immersed in a standard PBS buffer (pH 7.4) at 37°C; A working voltage is applied until a constant background current is reached, and the response current is measured at corresponding concentrations of glucose/sodium β-hydroxybutyrate, with the respective concentration being the following values: 2.2mM/1mM, 5mM/2mM, 10mM/3mM, 15mM/4mM, 20mM/6mM, 25mM/8mM, wherein the former represents the glucose concentration and the latter represents the sodium β-hydroxybutyrate concentration.

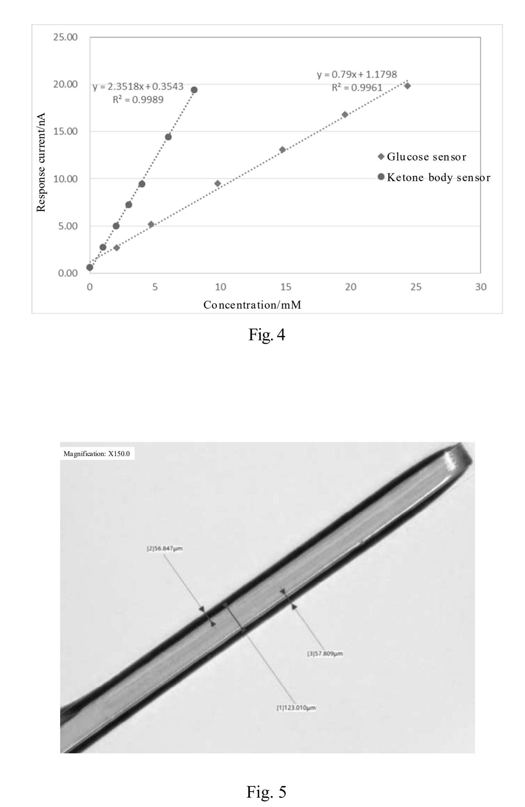

Fig.4 is a curve graph showing the response current-test concentration of Embodiment 1 of the blood glucose + blood ketone sensor according to the present disclosure. As shown in Fig.4, the dual-component sensor of Embodiment 1 shows good linearity in its response to glucose and sodium β-hydroxybutyrate. Specifically, as can be seen that the R2 values of the linear curves of the sensor's response currents to glucose and sodium β-hydroxybutyrate are both greater than 0.996, indicating a good linear relationship. The sensitivity of the glucose working electrode is 0.79nA/mM, and the sensitivity of the ketone body working electrode is 2.35nA/mM.

### Embodiment 2

Firstly, preparing a monitoring probe with dual working electrodes in front and rear, respectively referred to as the first working electrode and the second working electrode.

Secondly, the β-hydroxybutyrate sensitive layer solution and the glucose sensitive layer solution are prepared as the same formula as in Embodiment 1, wherein the metal polymer is a ruthenium-coordinated metal polymer. The glucose sensitive layer solution is deposited on the first working electrode, and the β-hydroxybutyrate sensitive layer solution is deposited on the second working electrode, both forming a linear pattern, wherein the area of the glucose sensing layer is approximately 0.3mm², and the area of the ketone sensing layer is approximately 0.27mm²; A dual-component responsive sensing layer electrode incorporating glucose and ketone bodies is obtained after curing.

Proceeding coating for the diffusion-limiting membrane, compatible membrane, and biocompatible membrane in the same manner as in Embodiment 1, the sensor of Embodiment 2 with an area ratio of blood glucose to blood ketone sensing layer of 1.11:1 is obtained.

Fig.5 is a photograph showing the monitoring probe of Embodiment 2 of the blood glucose + blood ketone sensor according to the present disclosure.

The sensor of Embodiment 2 is immersed in a standard PBS buffer (pH 7.4) at 37°C; A working voltage is applied until a constant background current is reached, and the response current is measured at corresponding concentrations of glucose/sodium β-hydroxybutyrate, with the respective concentration being the following values: 2.2mM/1mM, 5mM/2mM, 10mM/3mM, 15mM/4mM, 20mM/6mM, 25mM/8mM, the former represents the glucose concentration and the latter represents the sodium β-hydroxybutyrate concentration.

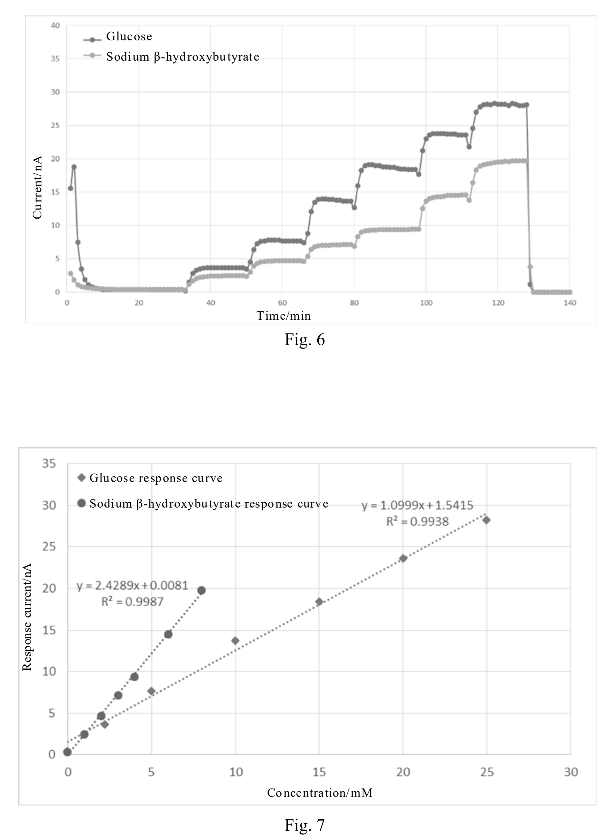

Fig.6 is a curve graph showing the response current-test time of Embodiment 2 of the blood glucose + blood ketone sensor according to the present disclosure. Fig.7 is a curve graph showing the response current-test concentration of Embodiment 2 of the blood glucose + blood ketone sensor according to the present disclosure. As shown in Fig.6 and Fig.7, the dual-component sensor of Embodiment 2 shows good linearity in its response to glucose and sodium β-hydroxybutyrate. Specifically, as shown in Fig.7, the R² values of the linear curves of the sensor's response currents to glucose and sodium β-hydroxybutyrate are both greater than 0.99, indicating a good linear relationship. The sensitivity of the glucose working electrode is 1.10nA/mM, and the sensitivity of the ketone body working electrode is 2.43nA/mM.

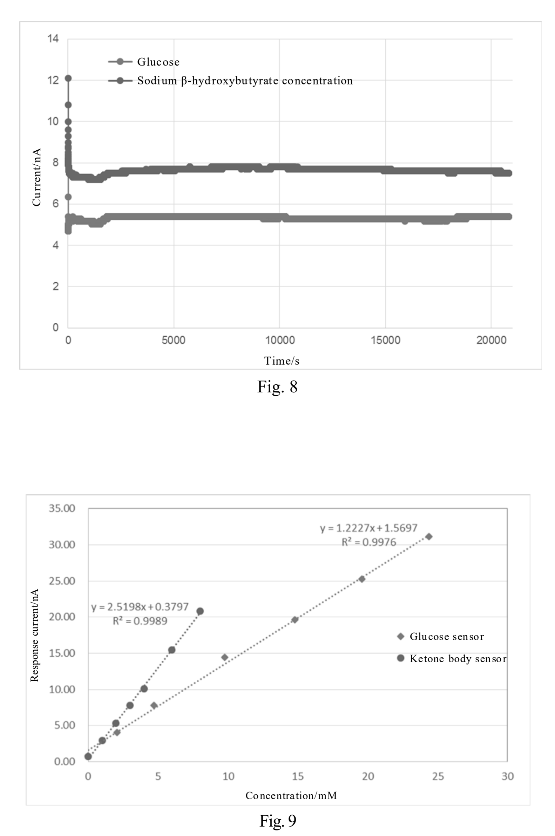

The sensor of Embodiment 2 is immersed in a standard PBS buffer (pH 7.4) at 37°C for 336 hours (14 days). Fig.8 is a graph showing the glucose/sodium β-hydroxybutyrate response current-time curve of Embodiment 2 of the blood glucose + blood ketone sensor related to the present disclosure. As shown in Fig.8, both current signals of the sensor remained stable during the test period from day 0 to day 14.

### Embodiment 3

A monitoring probe with dual working electrodes is prepared in the same manner as in Embodiment 1, the glucose sensitive layer solution is deposited on the first working electrode and the β-hydroxybutyrate sensitive layer solution is deposited on the second working electrode, both forming a linear pattern, wherein the area of the glucose sensing layer is approximately 0.39 mm², the area of the ketone sensing layer is approximately 0.26 mm²; That is the area ratio of the blood ketone to blood glucose sensing layers is 1:1.5; A dual-component responsive sensing layer electrode incorporating both glucose and ketone bodies is obtained After curing.

Subsequently, proceeding coating for the diffusion-limiting membrane, compatible membrane, and biocompatible membrane in the same manner as in Embodiment 1, the sensor of Embodiment 3 with an area ratio of blood glucose to blood ketone sensing layer of 1:1.5 is obtained.

The sensor of Embodiment 3 is immersed in a standard PBS buffer (pH 7.4) at 37°C; A working voltage is applied until a constant background current is reached, and the response current is measured at corresponding concentrations of glucose/sodium β-hydroxybutyrate, with the respective concentration being the following values: 2.2mM/1mM, 5mM/2mM, 10mM/3mM, 15mM/4mM, 20mM/6mM, 25mM/8mM, the former represents the glucose concentration and the latter represents the sodium β-hydroxybutyrate concentration.

Fig.9 is a curve graph showing the response current-test concentration of Embodiment 3 of the blood glucose + blood ketone sensor according to the present disclosure. As shown in Fig.9, the dual-component sensor of Embodiment 3 shows good linearity in its response to glucose and sodium β-hydroxybutyrate. Specifically, the R² values of the linear curves of the sensor's response currents to glucose and sodium β-hydroxybutyrate are both greater than 0.997, indicating a good linear relationship. The sensitivity of the glucose working electrode is 1.22nA/mM, and the sensitivity of the ketone body working electrode is 2.52nA/mM.

### Comparative Example 1

Firstly, preparing a monitoring probe with dual working electrodes in front and rear, respectively referred to as the first working electrode and the second working electrode.

Secondly, the β-hydroxybutyrate sensitive layer solution and the glucose sensitive layer solution are prepared as the same formula as in Embodiment 1, the glucose sensitive layer solution is deposited on the first working electrode, and the β-hydroxybutyrate sensitive layer solution is deposited on the second working electrode, both forming a linear pattern, wherein the area of the glucose sensing layer is approximately 0.3mm², and the area of the ketone sensing layer is approximately 0.45mm²; A dual-component responsive sensing layer electrode incorporating both glucose and ketone bodies is obtained after curing.

And then, the dual-component responsive electrode obtained as mentioned above is coated with the same three-layer formula as in Embodiment 1, a dual-component sensor with a membrane thickness of approximately 57 micrometers is ultimately obtained.

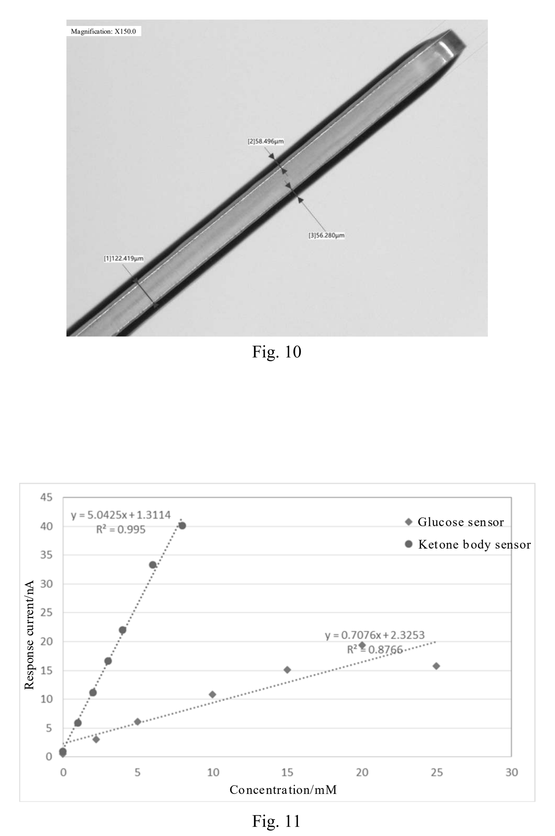

Fig.10 is a photograph showing the monitoring probe of Comparative Example 1 of the blood glucose + blood ketone sensor according to the present disclosure.

The sensor of Comparative Example 1 is immersed in a standard PBS buffer (pH 7.4) at 37°C; A working voltage is applied to the relative reference electrode until a constant background current is reached, and the response current is measured at corresponding concentrations of glucose/sodium β-hydroxybutyrate, with the respective concentration being the following values: 2.2mM/1mM, 5mM/2mM, 10mM/3mM, 15mM/4mM, 20mM/6mM, 25mM/8mM, the former represents the glucose concentration and the latter represents the sodium β-hydroxybutyrate concentration.

Fig.11 is a curve graph showing the response current-test concentration of Comparative Example 1 of the blood glucose + blood ketone sensor according to the present disclosure

The dual-component sensor in Comparative Example 1 shows good linearity in its current response to the concentration of sodium β-hydroxybutyrate, but shows poor current response to the concentration of glucose. Specifically, as shown in Fig.11, the R² values of the linear curves of the response current of the sensor to glucose and sodium β-hydroxybutyrate are 0.877 and 0.995 respectively, indicating that the glucose sensor does not has linearity in the range of 0-25mM. This may be because when the response concentration is high, the reaction volume of the ketone body side is even greater, and the product will affect the response of the working electrode of glucose side, thereby leading to the nonlinearity of glucose at high concentrations.

### Comparative Example 2

Firstly, preparing a monitoring probe with dual working electrodes in front and rear, respectively referred to as the first working electrode and the second working electrode

Secondly, the β-hydroxybutyrate sensitive layer solution and the glucose sensitive layer solution are prepared as the same formula as in Embodiment 1, the glucose sensitive layer solution is deposited on the first working electrode, and the β-hydroxybutyrate sensitive layer solution is deposited on the second working electrode, both forming a linear pattern, wherein the area of the glucose sensing layer is approximately 0.3mm², and the area of the ketone sensing layer is approximately 0.27mm²; A dual-component responsive sensing layer electrode incorporating both glucose and ketone bodies is obtained after curing.

And then, 80% ethanol-water solution is prepared as the solvent, an alcohol solution of poly(4-vinylpyridine-co-styrene) and PEGDGE is formulated at the corresponding concentrations according to Table 5 to obtain a membrane solution; the membrane solution is coated onto the electrode deposited with sensitive layer to form a dual-component monitoring working electrode incorporating a membrane layer of approximately 60 micrometers.

**Table 5. Composition of diffusion-limiting membrane solution**

| Substance | Concentration (mg/mL) |
|---|---|
| Polyvinyl pyridine | 100 |
| PDMS | 4 |
| PEGDGE | 35 |

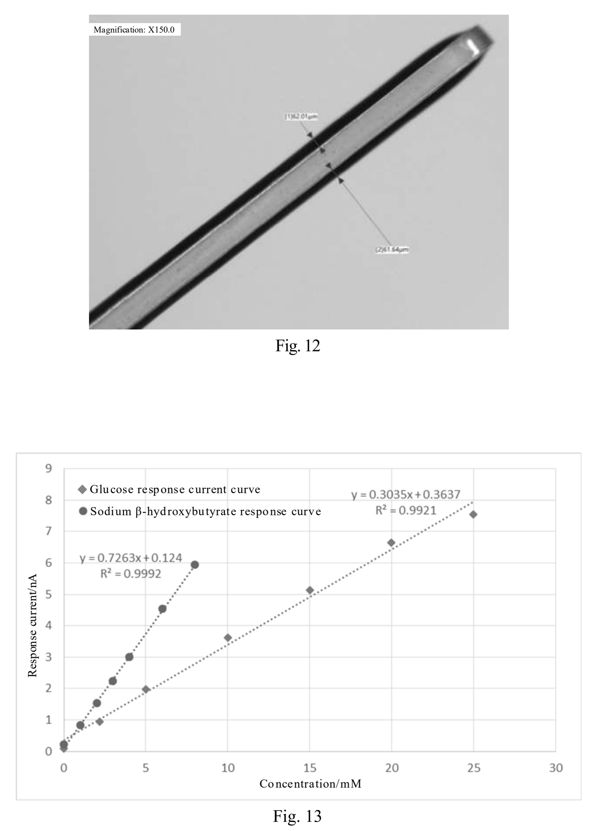

Fig.12 is a photograph showing the monitoring probe of Comparative Example 2 of the blood glucose + blood ketone sensor according to the present disclosure

The sensor of Comparative Example 2 is immersed in a standard PBS buffer (pH 7.4) at 37°C; A working voltage is applied to the relative reference electrode until a constant background current is reached, and the response current is measured at corresponding concentrations of glucose/sodium β-hydroxybutyrate, with the respective concentration being the following values: 2.2mM/1mM, 5mM/2mM, 10mM/3mM, 15mM/4mM, 20mM/6mM, 25mM/8mM, the former represents the glucose concentration and the latter represents the sodium β-hydroxybutyrate concentration.

Fig.13 is a curve graph showing the response current-test concentration of Comparative Example 2 of the blood glucose + blood ketone sensor according to the present disclosure. As shown in Fig.13, the dual-component sensor of Comparative Example 2 shows good linearity in its response to glucose and sodium β-hydroxybutyrate, Specifically, the R² values of the linear curves of the sensor's response currents to glucose and sodium β-hydroxybutyrate are both greater than 0.99, indicating a good linear relationship; Wherein, the sensitivity of the glucose working electrode is 0.30nA/mM, and the sensitivity of the ketone body working electrode is 0.73nA/mM. However, compared with Embodiment 2, the sensitivity of the electrodes in Comparative Example 2 is lower, indicating that the sensitivity of the electrodes can be significantly improved by adding the compatible membrane layer and the biocompatible membrane layer.

### Comparative Example 3

Firstly, preparing a monitoring probe with dual working electrodes in front and rear, respectively referred to as the first working electrode and the second working electrode.

Secondly, the β-hydroxybutyrate sensitive layer solution and the glucose sensitive layer solution are prepared as the same formula as in Embodiment 1, the glucose sensitive layer solution is deposited on the first working electrode, and the β-hydroxybutyrate sensitive layer solution is deposited on the second working electrode, both forming a linear pattern, wherein the area of the glucose sensing layer is approximately 0.3mm², and the area of the ketone sensing layer is approximately 0.27mm²; A dual-component responsive sensing layer electrode incorporating both glucose and ketone bodies is obtained after curing.

And then, ethanol is prepared as the solvent, an alcohol solution of polyvinyl pyridine and PEGDGE is formulated at the corresponding concentrations according to Table 2 to obtain a diffusion-limiting membrane solution. The diffusion-limiting membrane solution is coated onto the electrode deposited with the sensitive layer reagent to form a dual-component monitoring working electrode incorporating a membrane layer of approximately 25 micrometers.

**Table 6. Composition of diffusion-limiting membrane solution**

| Substance | Concentration (mg/mL) |
|---|---|
| Polyvinyl pyridine | 100 |
| PDMS | 4 |
| PEGDGE | 35 |

Lastly, Preparing an 80% ethanol-water solution, a solution of (poly(4-vinylpyridine-g-polyethylene glycol)-co-styrene) and PEGDGE is formulated at corresponding concentrations according to Table 7 to obtain a biocompatible membrane solution to obtain a biocompatible membrane solution; The biocompatible membrane solution is coated onto the diffusion-limiting membrane solution of the working electrode to form a working electrode incorporating a biocompatible membrane layer of approximately 25 micrometers, with a total membrane thickness of approximately 56 micrometers.

**Table 7. Composition of biocompatible membrane solution**

| Substance | Concentration (mg/mL) |
|---|---|
| (Poly4 - vinylpyridine -g- polyethylene glycol) - co - polystyrene | 100 |
| PEGDGE | 15 |

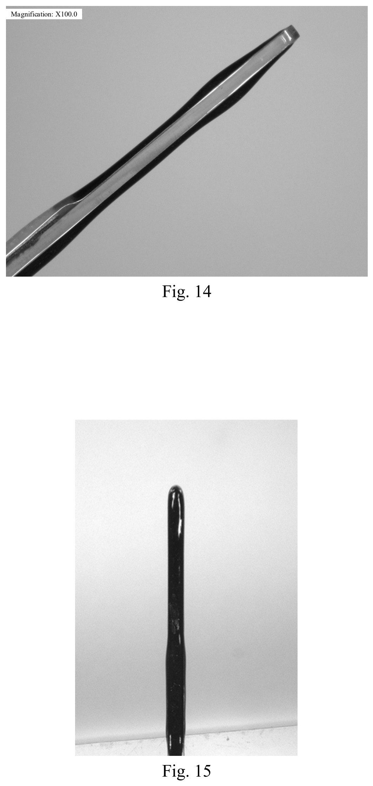

Fig.14 is a photograph showing the monitoring probe of Comparative Example 3 of the blood glucose + blood ketone sensor according to the present disclosure. Fig.15 is a photograph showing another view of the monitoring probe of Comparative Example 3 of the blood glucose + blood ketone sensor according to the present disclosur3.

As shown in Fig.14 and Fig.15, it can be seen that in the absence of a compatible membrane layer, the morphology of the semi-permeable membrane of the prepared sensor is wrinkled, with uneven thickness at the top and bottom, which is disadvantageous for preparing sensors with high consistency.

### [Blood Ketone Sensor]

### Embodiment 1, Comparative Examples 1-2

Firstly, preparing a monitoring probe with working electrodes.

Secondly, preparing the reagent materials for the sensitive layer of each blood ketone sensor example and comparative example according to Table 8 (ruthenium complex metal polymer, β-hydroxybutyrate dehydrogenase, diaphorase, NAD+, stabilizer, crosslinking agent, etc.). Each substance is dissolved in corresponding buffered solvents according to the concentration parameters provided in the table, ensuring complete dissolution via ultrasonic oscillation, a β-hydroxybutyrate sensitive layer solution is obtained for each embodiment.

The β-hydroxybutyrate sensitive layer solution is deposited onto the working electrode to form a linear pattern with a width of approximately 160 micrometers and a length of approximately 2.1 mm; an enzyme sensing layer is obtained after curing.

**Table 8. Embodiments of blood ketone sensor**

| Substance | Embodiment 1 | Comparative Example 1 | Comparative Example 2 |
|---|---|---|---|
| Metal polymer (mg/mL) | 10 | 10 | 10 |
| β-Hydroxybutyrate dehydrogenase (mg/mL) | 6 | 6 | 6 |
| Diaphorase (mg/mL) | 6 | 6 | 6 |
| NAD+ (mg/mL) | 6 | 6 | 6 |
| Proserum (mg/mL) | 6 | / | 6 |
| PEGDGE (mg/mL) | 5 | 5 | 5 |
| Solvent | HEPES buffer solution | HEPES buffer solution | PBS |

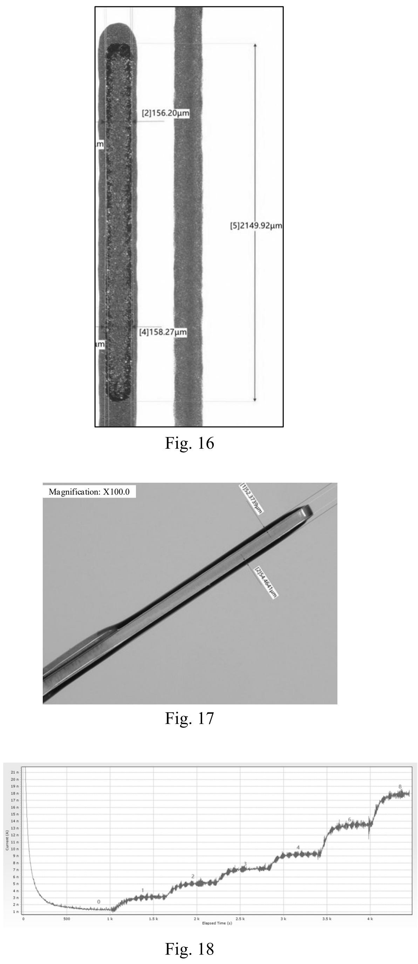

Fig.16 is a photograph showing the monitoring probe of the sensor in Embodiment 1, Comparative Examples 1-2 of the blood ketone sensor according to the present disclosure.

And then, pure ethanol is prepared as the solvent, an alcohol solution of polyvinyl pyridine, polydimethylsiloxane, and PEGDGE is formulated at the corresponding concentrations according to Table 9 to obtain a diffusion-limiting membrane solution. The diffusion-limiting membrane solution is coated onto the working electrode with the β-hydroxybutyrate sensitive layer solution deposited to form a working electrode incorporating a diffusion-limiting membrane layer of approximately 20 micrometers.

**Table 9. Composition of diffusion-limiting membrane solution**

| Substance | Concentration (mg/mL) |
|---|---|
| Polyvinyl pyridine | 92 |
| PDMS | 3 |
| PEGDGE | 30 |

Preparing an 80% ethanol-water solution, a solution of poly(4-vinylpyridine-co-styrene) and PEGDGE is formulated at the corresponding concentrations according to Table 10 to obtain a compatible membrane solution; The compatible membrane solution is coated onto the working electrode coated with the diffusion-limiting membrane layer to form a working electrode incorporating a compatible membrane layer of approximately 15 micrometers.

**Table 10. Composition of compatible membrane solution**

| Substance | Concentration (mg/mL) |
|---|---|
| Poly4 - vinyl pyridine - b - polystyrene | 70 |
| PEGDGE | 10 |

Preparing an 80% ethanol-water solution, a solution of (poly(4-vinylpyridine-g-polyethylene glycol)-co-styrene) and PEGDGE is formulated at corresponding concentrations according to Table 11 to obtain a biocompatible membrane solution; The biocompatible membrane solution is coated onto the working electrode coated with the compatible membrane layer to form a working electrode incorporating a biocompatible membrane layer of approximately 15 micrometers, with a total membrane thickness of approximately 50 micrometers.

**Table 11. Composition of biocompatible membrane solution**

| Substance | Concentration (mg/mL) |
|---|---|
| (Poly4 - vinylpyridine -g- polyethylene glycol) - co - polystyrene | 70 |
| PEGDGE | 10 |

Fig.17 is a photograph showing the monitoring probe coated with polymer membrane layer in Embodiment 1, Comparative Examples 1-2 of the blood ketone sensor according to the present disclosure.

Lastly, testing the linearity and stability of ketone body biosensors obtained from Embodiment 1, Comparative Examples 1-2.

### (1) Linearity Test:

The ketone body biosensor is immersed in a standard PBS buffer (pH 7.4) at 37°C; a working voltage is applied until the ketone body biosensor reaches a constant background current, the sodium β-hydroxybutyrate at concentrations of 0mM, 1mM, 2mM, 3mM, 4mM, 6mM, and 8mM are added into the solution to measure the linearity of the reaction, after each addition of sodium β-hydroxybutyrate, allowing the solution to equilibrate for 10 minutes, and continuously stir the solution during the measurement process to ensure a uniform concentration.

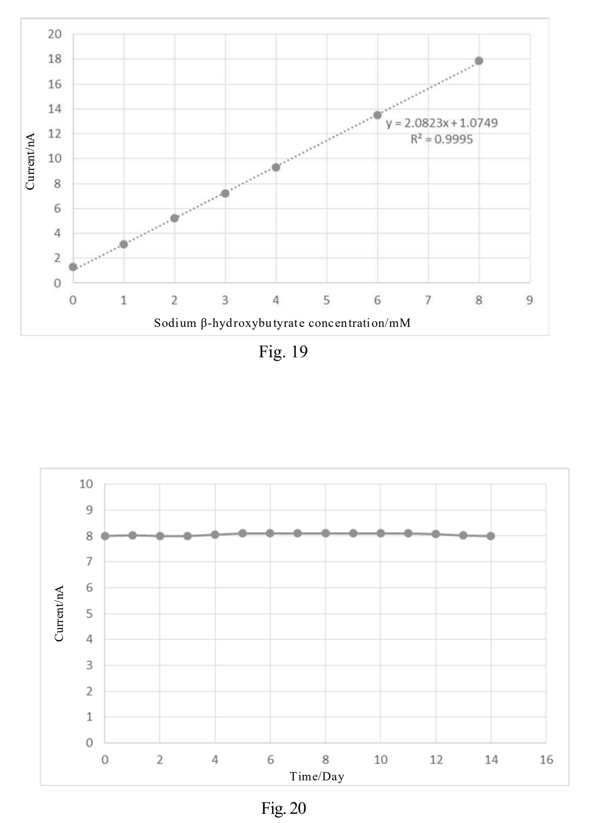

Fig.18 is a graph showing the response current-test concentration of the linear test of Embodiment 1 of the blood ketone sensor according to the present disclosure. Fig.19 is a curve graph showing the response current-test concentration of the linear test of Embodiment 1 of the blood ketone sensor according to the present disclosure.

As shown in Fig.18 and Fig.19, the ketone body biosensor of Embodiment 1 of blood ketone sensor shows good linearity in its response to glucose. Specifically, the glucose sensor in Embodiment 1 has a good linear correlation between the concentration of sodium β-hydroxybutyrate and the response current in the range of 0mM to 8mM.

### (2) Stability Test:

The ketone biosensor is immersed in 3mM sodium β-hydroxybutyrate (PBS buffer solution with pH 7.4 as a solvent) at 37°C for continuous measurement for 14 days.

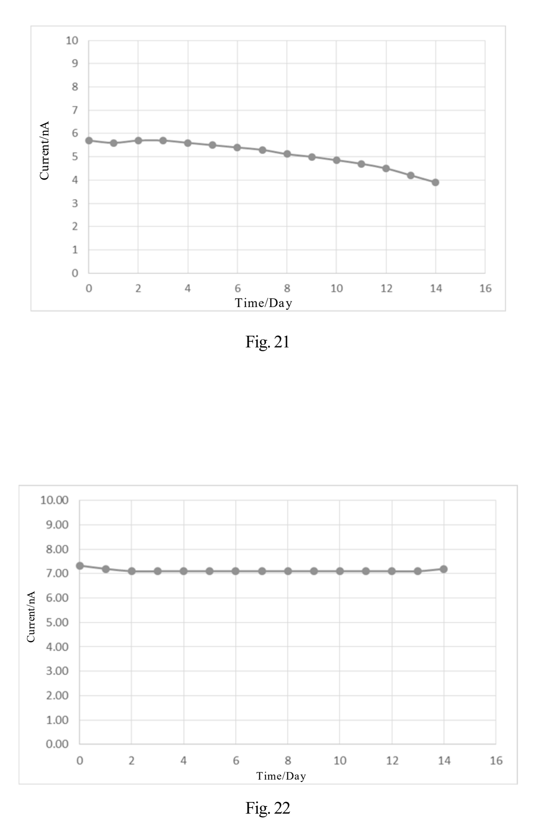

Fig.20 is a curve graph showing the response current-test concentration of the stability test of Embodiment 1 of the blood ketone sensor according to the present disclosure. Fig.21 is a curve graph showing the response current-test concentration of the stability test of Comparative Example 1 of the blood ketone sensor according to the present disclosure. Fig.22 is a curve graph showing the response current-test concentration of the stability test of Comparative Example 2 of the blood ketone sensor according to the present disclosure.

As shown in Figures 20-22, Comparative Example 1 fails to maintain stability for 14 days at 37°C, showing signs of performance degradation on the 5th day. However, Embodiment 1 and Comparative Example 2 can both maintain stability for 14 days due to the addition of stabilizers in their enzyme sensing layers, achieving longer-term stability for the ketone sensors. The response value of Embodiment 1 is higher than that of Comparative Example 2, indicating that using HEPES buffer solution as the solvent for the sensitive layer reagent is superior to using PBS buffer solution as the solvent for the sensitive layer reagent.

### Comparative Example 3

A monitoring probe with working electrode is prepared in the same manner as in Embodiment 1 of blood ketone, and the enzyme sensing layer is disposed on the working electrode to obtain a sensor without the semi-permeable membrane.

The sensor of Comparative Example 3 is exposed to a solution containing 1mM β-hydroxybutyrate sodium standard PBS buffer (pH 7.4) at 37°C for 1 hour.

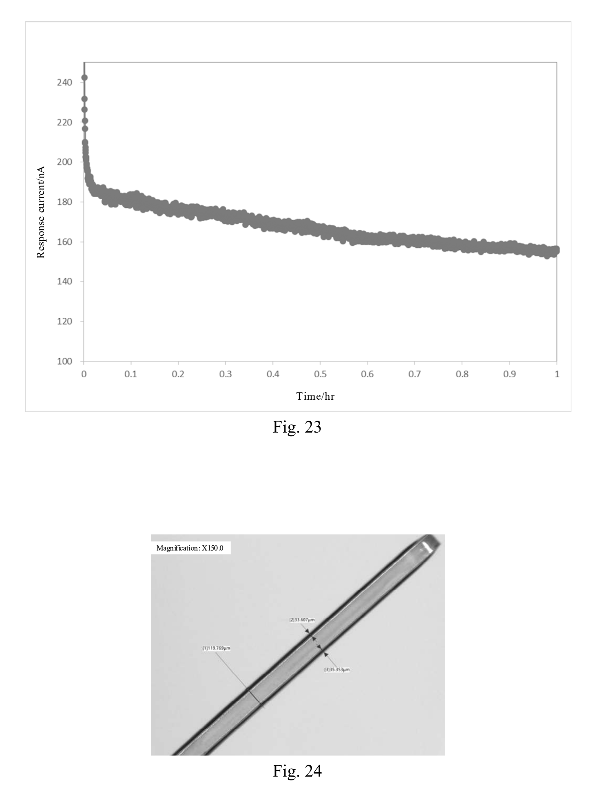

Fig.23 is a graph showing the response current-test concentration of the linear test of Comparative Example 3 of the blood ketone sensor according to the present disclosure.

As shown in Fig.23, during the 1-hour test time, the current signal generated by the sensor gradually decreased from 240nA to below 155nA, indicating poor stability. The test results indicate that the sensor without the semi-permeable membrane shows poor stability when testing the concentration of β-hydroxybutyrate sodium solution.

### Comparative Example 4

A monitoring probe with working electrode is prepared in the same manner as in Comparative Example 4 of blood ketone, and the enzyme sensing layer is disposed on the working electrode to obtain a sensor without the semi-permeable membrane.

Preparing an 80% ethanol-water solution, a solution of poly(4-vinylpyridine-co-styrene) and PEGDGE is formulated at the corresponding concentrations according to the below table to obtain an inner layer membrane solution; the inner layer membrane solution is coated onto the working electrode disposed with the enzyme sensing layer to form a working electrode incorporating a poly(4-vinylpyridine)-b-polystyrene membrane layer with a thickness of approximately 35 micrometers.

**Table 12. Composition of Inner layer membrane solution**

| Substance | Concentration (mg/mL) |
|---|---|
| Poly4 - vinyl pyridine - b - polystyrene | 70 |
| PEGDGE | 10 |

Fig.24 is a photograph showing the working electrode of Comparative Example 4 of the blood ketone sensor according to the present disclosureₒ

Preparing an 80% ethanol-water solution, A solution of (poly(4-vinylpyridine-g-polyethylene glycol)-co-styrene) and PEGDGE is formulated at corresponding concentrations according to Table 13 to obtain an outer layer membrane solution; the outer layer membrane solution is coated onto the working electrode coated with the inner layer membrane to form a working electrode incorporating an outer layer membrane of approximately 15 micrometers, with a total membrane thickness of approximately 50 micrometers.

**Table 13. Composition of outer layer membrane solution**

| Substance | Concentration (mg/mL) |
|---|---|
| (Poly4 - vinylpyridine -g- polyethylene glycol) - co - polystyrene | 70 |
| PEGDGE | 10 |

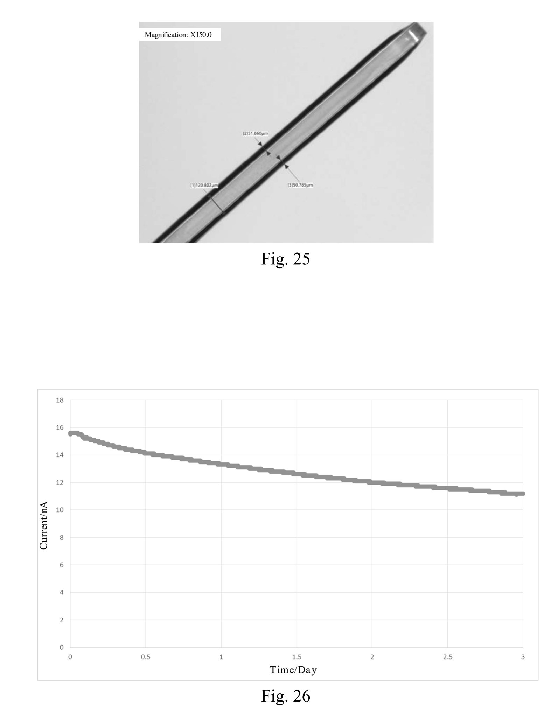

Fig.25 is a photograph showing the monitoring probe of the sensor of Comparative Example 4 of the blood ketone sensor according to the present disclosure.

Lastly, carry out stability test for the obtained ketone body sensor; that is the ketone biosensor of Comparative Example 4 is immersed in 3mM β-hydroxybutyrate sodium (PBS buffer solution with pH 7.4 as the solvent) at 37°C, the monitoring lasting 3 days.

Fig.26 is a curve graph showing the response current-test time of the sensor of Comparative Example 4 of the blood ketone sensor according to the present disclosure.

As shown in Fig.26, the initial response current of the sensor of Comparative Example 4 is 15.6nA, and after three days, the response current of the sensor decreases to 11.2nA, a total decrease of 28.2%, with an average daily decrease of 9.4% per day. This indicates that the sensor coated only with poly(4-vinylpyridine)-b-polystyrene and (poly(4-vinylpyridine-g-polyethylene glycol))-copolystyrene membranes has a poor stability.

The main reason is that compared to Embodiment 1 of blood ketone, the membrane layers combination has a higher permeability to β-hydroxybutyrate sodium, indicating the diffusion limitation effect of the membranes are not strong, and unable to reduce the permeability of β-hydroxybutyrate sodium and limit the escape of small molecules from the enzyme layer, resulting in poor stability.

### Comparative Sample 5

Firstly, preparing a monitoring probe with working electrodes.

Preparing corresponding reagent according to the same enzyme layer formula as in Embodiment 1 of blood ketone, an ultra-micro pipetting robot is used to drop-coat β-hydroxybutyrate sensitive layer solution onto the working electrode to form a pattern with six independent dots, the dot diameter is 150 micrometers; an enzyme sensor with six-dot pattern is obtained after curing.

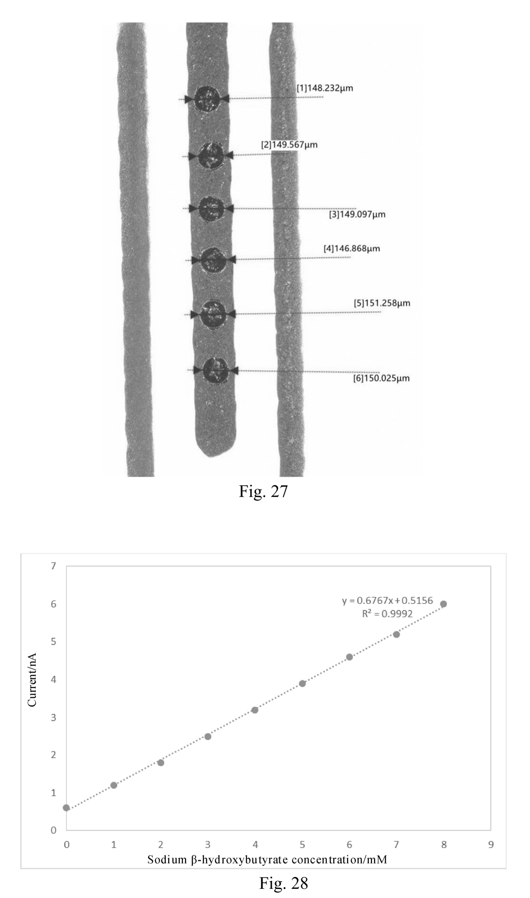

Fig.27 is an image showing the working electrode of the sensor of Comparative Example 5 of the blood ketone sensor according to the present disclosure

And then, the 6-point pattern enzyme sensor is coated according to the same coating scheme as in embodiment 1 of blood ketone to obtain the ketone body sensor of Comparative Example 5.

Lastly, carry out linearity test for the obtained ketone sensor of Comparative Example 5: That is the ketone body biosensor is immersed in a standard PBS buffer (pH 7.4) at 37°C; A working voltage is applied until the ketone body biosensor reaches a constant background current, and the sodium β-hydroxybutyrate at concentrations of 1mM, 2mM, 3mM, 4mM, 6mM, 7mM and 8mM are added into the solution to measure the linearity of the reaction, after each addition of sodium β-hydroxybutyrate, and continuously stir the solution during the measurement process to ensure a uniform concentration.

Fig.28 is a graph showing the response current-test concentration of the linear test of Comparative Example 5 of the blood ketone sensor according to the present disclosure

As shown in Fig.28, as can be seen that the blood ketone sensor shows good linearity in its response to sodium β-hydroxybutyrate within the concentration range of 0-8 mM, but its response sensitivity is lower, that is, the slope of the linear fitting curve is 0.677 nA/mM. However, the sensitivity of the sensor in Embodiment 1 is 2.08 nA/mM, while the sensitivity of Comparative Example 5 is only 32.5% of that in Embodiment 1. This is caused by smaller area of the enzyme layer in Comparative Example 5, and low reaction area of sodium β-hydroxybutyrate permeating through the membrane layer. This indicates that increasing the area of the enzyme layer may effectively increase the sensitivity of the sensor.

Various examples of the present disclosure have been described in the specific embodiments above. Although these descriptions directly refer to the examples mentioned above, it should be understood that a person skilled in the art may conceive of modifications and/or variations to the specific examples shown and described in the present disclosure. Any such modifications or variations falling within the scope of this specification are also intended to be included. Unless specifically stated, it is the intention of the inventor that the terms in the specification and claims be given their ordinary and customary meanings by those skilled in the art.

## Claims

1. A multi-analyte monitoring sensor, **characterized in that**,
The sensor includes a substrate, a working electrode disposed on the substrate, an enzyme sensing layer disposed on the working electrode, and a polymer membrane layer disposed on the enzyme sensing layer.
The working electrode includes a first working electrode and a second working electrode;
The enzyme sensing layer includes a first analyte enzyme layer and a second analyte enzyme layer, the first analyte enzyme layer is disposed on the first working electrode and the second analyte enzyme layer is disposed on the second working electrode, the first analyte is glucose, and the second analyte is ketone.
The polymer membrane layer is permeable to both the first analyte and the second analyte and covers the first analyte enzyme layer and the second analyte enzyme layer.

2. The multi-analyte monitoring sensor according to claim 1, **characterized in that**,
The polymer membrane layer includes a vinyl pyridine-based polymer, a crosslinking agent, and a modifier, wherein the mass fraction of the vinyl pyridine-based polymer in the polymer membrane layer is 80% to 100%.

3. The multi-analyte monitoring sensor according to claim 1, **characterized in that**,
The polymer membrane layer successively includes a first membrane layer disposed on the enzyme sensing layer, a transition layer formed on the first membrane layer, and a biocompatible second membrane layer formed on the transition layer.

4. The multi-analyte monitoring sensor according to claim 3, **characterized in that**,
The first membrane layer is formed by a first type of polymer, the second membrane layer is formed by a second type of polymer, the transition layer is formed by a third type of polymer, wherein the third type of polymer is a copolymer formed by a first monomer identical or similar to the first type of polymer and a second monomer identical or similar to the second type of polymer.

5. The multi-analyte monitoring sensor according to claim 3 or claim 4, **characterized in that**,
The first membrane layer includes a vinyl pyridine-based polymer, the transition layer and the second membrane layer include a copolymer of vinyl pyridine and styrene.

6. The multi-analyte monitoring sensor according to claim 1, **characterized in that**,
The area of the first analyte enzyme layer is greater than the area of the second analyte enzyme layer.

7. The multi-analyte monitoring sensor according to claim 1, **characterized in that**,
The area ratio of the first analyte enzyme layer to the second analyte enzyme layer is 1:1 to 1.5:1.

8. The multi-analyte monitoring sensor according to claim 1, **characterized in that**,
The first analyte enzyme layer and/or the second analyte enzyme layer are/is in a linear shape.

9. The multi-analyte monitoring sensor according to claim 1, **characterized in that**,
The second analyte enzyme layer includes hydroxybutyrate dehydrogenase, diaphorase, coenzyme, and an electron mediator, in the second analyte enzyme layer, the mass fraction of hydroxybutyrate dehydrogenase is 10% to 20%, the mass fraction of diaphorase is 5% to 20%, the mass fraction of coenzyme is 10% to 30%, and the mass fraction of the electron mediator is 10% to 30%.

10. The multi-analyte monitoring sensor according to claim 1, **characterized in that**,
The sensor further includes a reference electrode and a counter electrode, wherein the first working electrode and the second working electrode are respectively positioned on opposite sides of the substrate, and the reference electrode and the counter electrode are respectively positioned on both sides of the substrate.
